# EUROPEAN PATENT SPECIFICATION

(11) **EP 0 839 516 B1**
(45) Date of publication and mention of the grant of the patent: **19.03.2003**
(21) Application number: 97203352.6
(22) Date of filing: 29.10.1997
(51) Int. Cl.: A61K 7/16

(54) **Triglyceride containing Toothpaste**
Triglycerid enthaltende Zahnpasta
Pâte dentifrice contenant un triglycéride

(30) Priority: 30.10.1996 NL 1004384
(43) Date of publication of application: 06.05.1998
(73) Proprietor: Sara Lee/DE N.V., 3532 AA Utrecht (NL)
(72) Inventor: Venema, Franciscus Ties, 3781 XJ Voorthuizen (NL); Brinkman, Jan Anton, 1212 CD Hilversum (NL)
(74) Representative: Smulders, Theodorus A.H.J., Ir.

(56) References cited:
- EP-A- 0 272 878
- EP-A- 0 566 174
- WO-A-95/32637
- US-A- 4 844 883
- US-A- 5 401 496

## Description

The present invention relates to a dentifrice, after the use of which the teeth have a smooth and pleasant feel, while, furthermore, the adhesion of bacteria and plaque can be less.

Dentifrices are understood to be those products which, when used normally, are hardly, if at all, swallowed but remain in the oral cavity for some time, thus coming into intensive contact with nearly all the dental faces. Examples of such products are toothpastes, tooth polishes, chewing gum, mouthwashes and mouth powders. The invention relates to a toothpaste.

Regular brushing and care of the oral cavity using a suitable product such as a toothpaste or a mouthwash is effective in preventing rapid plaque formation and the incidence of the other disorders, but even regular brushing is not sufficient to completely remove plaque and inhibit caries.

In the care of the oral cavity, it is desired that afterwards the teeth should have a smooth and pleasant feel. This is not only a pleasant sensation but may also have the advantage that less adhesion of plaque and/or bacteria occurs. This is of course important to inhibit caries.

Patent document EP-A-0 272 878 discloses the use of 0.01 to 5 wt.% of triglyceride of one or more C₈-C₁₀ fatty acid moieties in dentifrices for improving the sensory perception in the oral cavity.

Patent document US-A-5 401 496 discloses a toothpaste containing 64 % of capric/caprylic triglyceride along with polishing agents and its use for plaque removal.

Surprisingly, it has been found that the use of a triglyceride in an amount of 0.1 to 12.5 wt.%, more in particular of 7.5 to 12.5 wt.%, in toothpastes has an excellent effect in the care of the oral cavity, more in particular to obtain the intended effect.

In general, according to the invention, different triglycerides of saturated and/or unsaturated fatty acids can be used, but it is preferred to use fatty acid moieties having a chain length of at least 6 carbon atoms, e.g. 6-14 carbon atoms, more in particular one or more chains selected from C₈, C₁₀ and combinations thereof. Accordingly, capryl and caprylyl moieties are preferred.

The invention therefore relates to a toothpaste comprising an active amount of fatty acid triglycerides, as well as conventional additives.

As conventional additives, those conventional for dentifrices can be used. Examples thereof are, inter alia, fluoride-providing compounds, antibacterial agents, polishing agents, carrier, humectant, thickener, odors and flavors, anti-tartar agents, agents for inhibiting the sensitivity of teeth, wound-healing agents, as well as activity-increasing agents, such as the different polymers. It may also be useful to include hydrogen peroxide-producing enzyme systems in the dentifrices.

To obtain an antibacterial effect, known antibacterial agents can be used, such as xylitol, a diphenyl ether or a phenol derivative such as triclosan or bromochlorophene. It is also possible, however, to use one of the other antibacterial agents. More in particular, it can be considered to use a lantibiotic such as nisin, optionally in combination with triclosan or one of the other stated components.

The presence of fluoride-producing agents in the dentifrices according to the invention is important. The use of fluoride ions or fluorine ions-providing compounds as anti-caries agent is known. Suitable compounds can be completely or a little water-soluble. They are characterized by the capacity to release fluoride ions in water and by the absence of undesirable reactions with other components of the formulation. Suitable compounds are the inorganic fluoride salts such as water-soluble alkali and alkaline earth metal salts, e.g. sodium fluoride, potassium fluoride, barium fluoride, calcium fluoride, ammonium fluoride, copper fluoride or zinc fluoride, as well as sodium fluorosilicate, ammonium fluorosilicate, sodium fluorozirconate, sodium monofluorophosphate, aluminum mono- and difluorophosphate and fluorine-containing sodium calcium pyrophosphate. Alkali metal and tin fluoride such as sodium fluoride and tin(II) fluoride, sodium monofluorophosphate, organic fluorides such as amino fluorides and mixtures of such compounds are preferred.

The amount of fluoride-providing compound depends to some extent on the nature of the compounds, the solubility and the nature of the dentifrice. However, it should be a non-toxic, active amount. In toothpastes, such amount will generally be between 0.005 and 3.0%, which amount is capable of providing about 5,000 ppm fluorine ions. A suitable minimum amount is preferably 300 ppm. In general, it is preferred to use an amount leading to the presence of 300 to about 2000, more in particular 800 to about 1500 ppm fluoride ions in the dentifrice. If alkali metal fluorides and tin fluorides are used, the fluoride compound is present in an amount of up to about 2 wt.%, more in particular 0.5 to 1 wt.%. Sodium monofluorophosphate may be present in an amount of about 0.1 to 3%.

The toothpaste also contains a polishing agent. Conventional polishing agents are water-insoluble sodium metaphosphate, potassium metaphosphate, tricalcium phosphate, dehydrated calcium phosphate, anhydrous dicalcium phosphate, calcium pyrophosphate, magnesium orthophosphate, trimagnesium phosphate, aluminum oxide, aluminum oxide hydrate, calcium carbonate, aluminum silicate, zirconium silicate, silica, bentonite and mixtures of two or more of these polishing agents. Other suitable polishing materials comprise the particulate thermosetting resins such as melamine phenol, and urea formaldehydes, as well as cross-linked polyepoxides and polyesters. Preferred polishing agents comprise silica having a particle size up to about 7 µm, an average particle size of about 1.1 µm and a surface area of maximally 5 cm²/g, silica gel or colloidal silica, as well as complex amorphous alkali metal aluminosilicate.

The polishing agent is present in the toothpaste in amounts of 10-99 wt.%. Preferably, however, this is 10-75 wt.%

In a toothpaste the liquid carrier will generally contain water and a humectant in amounts of 10-90 wt.%. Glycerin, propylene glycol, sorbitol, polypropylene glycol and/or polyethylene glycol are examples of suitable humectants/carriers. It may also be advantageous to use liquid mixtures of water, glycerin and sorbitol.

It is known to add phosphate compounds to dentifrices. Known inorganic phosphate compounds are trimetaphosphate and calcium phosphate. It is also known to add organic phosphate compounds to dentifrices. These agents strongly adhere to the dental enamel, thus reducing the surface tension. This is supposed to decrease the adhesion of plaque to the dental enamel. Preferably, polyphosphates are used, such as linear, tri- and/or tetrapolyphophates, most preferably in amounts of up to about 15 wt.%, based on the weight of the dentifrice.

Toothpastes generally contain a natural or synthetic thickener of gelling agent in amounts of about 0.1 to 10, preferably about 0.5 to about 5 wt.%. A suitable thickener is synthetic hectorite, a synthetic colloidal magnesium alkali metal silicate complex clay, which is available, e.g., as Laponite (e.g. CP, SP 2002, D) of Laporte Industries Limited.

Other suitable thickeners comprise irish moss, gum tragacanth, starch, polyvinylpyrrolidone, hydroxyethylpropyl cellulose, hydroxybutylmethyl cellulose, hydroxypropylmethyl cellulose, hydroxyethyl cellulose (e.g. Natrosol), sodium carboxymethyl cellulose and colloidal silica.

Suitable flavors or sweeteners may also be used. Examples of suitable flavor components are the flavor oils such as spearmint, peppermint oil, wintergreen, sassafras, clove, sage, eucalyptus, marjoram, lemon, orange and methyl silicate. Suitable sweeteners comprise sucrose, lactose, maltose, sorbitol, sodium cyclamate, perillartine, aspartame, saccharin and the like. The amount of the combined flavors and sweeteners together is preferably 0.1-5 wt.% of the formulation.

The dentifrices according to the invention may contain components that may reduce the sensitivity of teeth, such as specific alkali metal salts (strontium salts and/or potassium nitrate and/or potassium citrate). They may also contain wound-healing agents, such as allantoin, chlorophyll, tocopherol and herb extracts. Activity-increasing agents, i.e. agents prolonging the residence time of the active components in the oral cavity, are in particular all kinds of polymers such as Gantrez and phophonates.

According to a preferred embodiment the dentifrices also comprise an enzyme system as described in U.S. patents 4,150,113 and 4,871,532. The hydrogen peroxide-producing enzyme systems described therein also have an advantageous effect on the reduction of the bacterial activity in the oral cavity. The most effective enzyme is the oxidoreductase enzyme glucose-oxidase. This enzyme may be present in combination with other enzymes such as amyloglucosidase, dextranase and/or mutanase, optionally in combination with zinc ion-providing compounds and/or 8-hydroxyquinoline derivatives.

Other enzymes such as lactoperoxidase, lactoferrin and lysozyme may also be present and provide a dentifrice with attractive supplementary properties.

The dentifrices may also contain surfactants, more in particular nonionic surfactants, although anionic surfactants may also be used. Examples of such surfactants that may be present in amounts of between 0.01 and 5 wt.% are, inter alia, ethoxylated alcohols and the like.

The manufacture of the dentifrices according to the invention comprises the conventional methods which are known to those skilled in the art.

The invention will now be illustrated by some non-limitative examples.

### EXAMPLES

A toothpaste was manufactured by a method conventional for toothpastes. The compositions thereof were as follows.

| Component | Wt.% | | | |
|---|---|---|---|---|
| silica | 20 | 20 | 20 | 20 |
| sodium fluoride | 0.24 | 0.24 | 0.24 | 0.24 |
| MFP | 0.28 | 0.28 | 0.28 | 0.28 |
| sorbitol | 20 | 20 | 20 | 20 |
| glycerol | 2 | 5 | 5 | 5 |
| propylene glycol | 3 | - | - | - |
| polyphosphate | - | 5 | 5 | - |
| capryl(yl) triglyceride | 10 | 5 | 10 | 5 |
| titanium dioxide | 0.5 | 0.5 | 0.5 | 0.5 |
| phosphate buffer | 1 | 1 | 1 | 1 |
| CMC | 1.7 | 1.7 | 1.7 | 1.7 |
| α-olefin sulfonate | 1.5 | 1.5 | 1.5 | 1.5 |
| water up to | 100 | 100 | 100 | 100 |

## Claims

1. Use of 0.1 - 12.5 wt.% of triglyceride of one or more fatty acid moieties in combination with 10-99 wt.% of polishing agent in a toothpaste for providing a tooth surface having a smooth feel after care of the oral cavity.

2. Use according to claim 1, wherein the amount of triglyceride ranges between 7.5 and 12.5 wt.%.

3. Use according to any of the claims 1-2, wherein as triglyceride a triglyceride of at least one fatty acid having at least 6 carbon atoms, preferably 6 to 14 carbon atoms, is used.

4. Use according to any of the claims 1-3, wherein a triglyceride of caprylic acid and/or caprylylic acid is used.

5. Use according to any of the claim 1-4, wherein the toothpaste comprises an abrasive, a thickener, as well as an aqueous carrier.

6. Use according to any of the claims 1-5, wherein the toothpaste contains as additives one or more of the components from the group consisting of water, antibacterial agents, fluoride-providing compounds, foaming agents, enzymes, colors, flavors and humectants.

7. A toothpaste comprising 7.6 to 12.5 wt % of a triglyceride of at least one fatty acid having at least 6 carbon atoms, preferably 6 to 14 carbon atoms and 10-99 wt.% of polishing agent.

8. A toothpaste according to claim 7, comprising one or more additives selected from the group consisting of abrasives, thickeners, aqueous carriers, antibacterial agents, fluoride-providing compounds, foaming agents, enzymes, colors, flavors and humectants.

9. A toothpaste according to claim 7 or 8, wherein a triglyceride of caprylic acid and/or caprylylic acid is present.

## Patentansprüche

1. Verwendung von 0,1 - 12,5 Gew.-% eines Triglycerids von einer oder mehr Fettsäureeinheiten in Kombination mit 10-99 Gew.-% Poliermittel in einer Zahnpasta zur Erzielung einer Zahnoberfläche mit einem glatten Gefühl nach Pflege der Mundhöhle.

2. Verwendung nach Anspruch 1, wobei die Menge des Triglycerids im Bereich zwischen 7,5 und 12,5 Gew.-% liegt.

3. Verwendung nach einem der Ansprüche 1 bis 2, wobei als Triglycerid ein Triglycerid von mindestens einer Fettsäure mit mindestens 6 Kohlenstoffatomen, bevorzugt 6 bis 14 Kohlenstoffatomen, verwendet wird.

4. Verwendung nach einem der Ansprüche 1 bis 3, wobei ein Triglycerid von Caprylsäure und/oder Caprylyisäure verwendet wird.

5. Verwendung nach einem der Ansprüche 1 bis 4, wobei die Zahnpasta einen Abrasivstoff, einen Verdicker und einen wässrigen Träger umfasst.

6. Verwendung nach einem der Ansprüche 1 bis 5, wobei die Zahnpasta als Additive eine oder mehr Komponenten aus der aus Wasser, antibakteriellen Mitteln, Fluorid-bereitstellenden Verbindungen, Schäumungsmitteln, Enzymen, Farbstoffen, Geschmacksstoffen und Feuchthattemitteln bestehenden Gruppe enthält.

7. Zahnpasta, umfassend 7,5 bis 12,5 Gew.-% eines Triglycerids von mindestens einer Fettsäure mit mindestens 6 Kohlenstoffatomen, bevorzugt 6 bis 14 Kohlenstoffatomen, und 10-99 Gew.-% Poliermittel.

8. Zahnpasta nach Anspruch 7, umfassend ein oder mehr Additive, ausgewählt aus der aus Abrasivstoffen, Verdickern, wässrigen Trägern, antibakteriellen Mitteln, Fluorid-bereltstellenden Verbindungen, Schäumungsmitteln, Enzymen, Farbstoffen, Geschmacksstoffen, und Feuchthaltemitteln bestehenden Gruppe.

9. Zahnpasta nach Anspruch 7 oder 8, in der ein Triglycerid von Caprylsäure und/oder Caprylylsäure vorliegt.

## Revendications

1. Utilisation de 0,1 à 12,5% en poids de triglycéride d'un ou de plusieurs fragments d'acide gras en association avec de 10 à 99% en poids d'un agent de polissage dans une pâte dentifrice pour fournir une surface dentaire conférant une impression de douceur après le soin de la cavité buccale.

2. Utilisation selon la revendication 1, dans laquelle la quantité de triglycéride varie dans la plage de valeurs comprises entre 7,5 et 12,5% en poids.

3. Utilisation selon l'une des revendications 1 et 2, dans laquelle, en tant que triglycéride, on utilise un triglycéride d'au moins un acide gras ayant au moins 6 atomes de carbone, de préférence de 6 à 14 atomes de carbone.

4. Utilisation selon l'une quelconque des revendications 1 à 3, dans laquelle on utilise un triglycéride d'acide caprylique et/ou d'acide caprylylique.

5. Utilisation selon l'une quelconque des revendications 1 à 4, dans laquelle la pâte dentifrice comprend un abrasif, un épaississant de même qu'un excipient aqueux.

6. Utilisation selon l'une quelconque des revendications 1 à 5, dans laquelle la pâte dentifrice contient en tant qu'additifs un ou plusieurs composants choisis dans le groupe constitué par l'eau, les agents antibactériens, les composés fournissant le fluorure, les agents moussants, les enzymes, les colorants, les arômes et les humectants.

7. Pâte dentifrice comprenant de 7,5 à 12,5% en poids d'un triglycéride d'au moins un acide gras ayant au moins 6 atomes de carbone, de préférence de 6 à 14 atomes de carbone et de 10 à 99% en poids d'un agent de polissage.

8. Pâte dentifrice selon la revendication 7, comprenant un ou plusieurs composants choisis dans le groupe constitué par les abrasifs, les épaississants, les excipients aqueux, les agents antibactériens, les composés fournissant le fluorure, les agents moussants, les enzymes, les colorants, les arômes et les humectants.

9. Pâte dentifrice selon la revendication 7 ou 8, dans laquelle un triglycéride d'acide caprylique et/ou d'acide caprylylique est présent.
